Europäisches Patentamt

European Patent Office

Office européen des brevets

(19)

(11) **EP 1 275 344 A1**

(12) **DEMANDE DE BREVET EUROPEEN**

(43) Date de publication:
**15.01.2003 Bulletin 2003/03**

(51) Int Cl.⁷: **A61B 17/11, A61F 2/06**

(21) Numéro de dépôt: **01116534.7**

(22) Date de dépôt: **09.07.2001**

(84) Etats contractants désignés:
**AT BE CH CY DE DK ES FI FR GB GR IE IT LI LU
MC NL PT SE TR**
Etats d'extension désignés:
**AL LT LV MK RO SI**

(71) Demandeurs:
• **Bioring SA**
**1027 Lonay (CH)**
• **Frey, Peter**
**1066 Epalinges (CH)**

(72) Inventeurs:
• **Andrieu, Raymond**
**1137 Yens (CH)**
• **Frey, Peter**
**1066 Epalinges (CH)**
• **Le Goff, Philippe**
**1052 Le Mont sur Lausanne (CH)**

(74) Mandataire: **Micheli & Cie**
**Rue de Genève 122,**
**Case Postale 61**
**1226 Genève-Thonex (CH)**

(54) **Dispositif pour l'anastomose, la maintenance et la protection vasculaire**

(57) La présente invention a pour objet un dispositif pour la connexion et le maintien et/ou la protection des conduits des systèmes circulatoires comportant un élément de forme cylindrique ou conique évidé (1) en un matériau résorbable (bioabsorbable ou biodégradable). Il comprend des organes de maintien (2, 3, 4) destinés à maintenir sans suture les extrémités des conduits des systèmes circulatoires à traiter sur ledit dispositif.

Fig.2

Fig.4

EP 1 275 344 A1

## Description

La présente invention concerne un dispositif de forme cylindrique ou conique, et en un matériau résorbable, destiné à servir à la connexion et au maintien et/ou à la protection des conduits circulatoires lors d'une intervention chirurgicale.

Le terme résorbable sera utilisé dans la suite du document pour définir de façon indifférenciée les caractéristiques d'un matériau bioabsorbable ou biodégradable.

Le terme conduit circulatoire signifie des conduits de tous types des systèmes circulatoires, par exemple sanguin, intestinal ou urinaire.

L'un des gestes les plus courants de la chirurgie cardiovasculaire actuelle consiste à pratiquer une suture termino - terminale ou une suture latéro - terminale entre les deux extrémités d'un conduit artériel ayant nécessité un sectionnement pour les besoins d'une intervention chirurgicale. Le résultat de ce geste chirurgical, et en général le geste lui-même, est appelé anastomose.

Dans la mesure où ce geste intervient le plus souvent sur des patients présentant un mauvais état vasculaire, il convient dans la majorité des cas d'exécuter la réparation artérielle dans un minimum de temps afin de ne pas prolonger à la fois les utilisations de systèmes circulatoires périphériques ou extra - corporels et/ou la durée de l'anesthésie pratiquée sur le patient. D'ailleurs, le chirurgien est souvent amené à réaliser la suture réparatrice par un surjet (consistant à utiliser un fil unique pour la mise en place d'une succession de points) de préférence à une technique de suture point par point. S'il est clair que le choix de la technique du surjet apporte au chirurgien un raccourcissement important du temps d'exécution, il est tout aussi exact que le résultat perd en reproductibilité par rapport à celui d'une suture point par point et est notamment souvent moins favorable. Ceci parce qu'un surjet a tendance à favoriser un rétrécissement du passage libre dans le conduit artériel provoquant un trouble circulatoire au voisinage de la suture et éventuellement, dans les cas extrêmes, même l'apparition d'une thrombose.

Considérant que ce sont plus de 1,9 millions d'interventions cardiovasculaires qui sont projetées à l'horizon de l'an 2002 pour la seule zone Europe ( *Sources : European Heart Institute*, *Cardiovascular Device Update (Aug. '98 page 2)* ) et que celles-ci s'accompagnent de façon quasi systématique du geste décrit ci-dessus, il apparaît clairement que l'amélioration de la technique concerne une majorité des praticiens. Une standardisation de l'anastomose accompagnée notamment d'un raccourcissement du temps d'exécution pour un résultat toujours comparable à celui d'une suture point par point pourrait améliorer considérablement le confort du praticien et la sécurisation de l'intervention, et contribuerait par conséquent à une diminution du risque lors de l'intervention chirurgicale cardiovasculaire en général.

Dans cette optique, on a tenté la mise au point de dispositifs et/ou techniques pour éviter une suture des extrémités distales et proximales lors de l'anastomose vasculaire. Ces dispositifs sont de forme tubulaire et en un matériau résorbable et permettent de raccourcir notablement la durée de l'intervention, mais ne donnent pas entière satisfaction vis à vis des suites post - implantation de sorte que cette technique n'a pas été poursuivie et que les techniques conventionnelles de suture sont encore dominantes dans le traitement des anastomoses.

L'utilisation du dispositif selon la présente invention consiste à enfiler les deux extrémités des conduits circulatoires à connecter sur le dispositif cylindrique ou conique de manière à ce que les deux extrémités soient mises en contact, et à maintenir les extrémités des conduits autour du dispositif par l'intermédiaire d'un système particulier, l'ensemble de cette procédure est décrit en détail dans la suite du document.

L'apport du dispositif dans le domaine de la chirurgie cardiovasculaire réparatrice est principalement qu'il supprime le besoin de sutures de toute sorte lors d'une re-connexion d'un conduit circulatoire sectionné pour les besoins d'une intervention. Les risques d'apparition de bourrelets internes au conduit, créant une contrainte dans l'écoulement du fluide et susceptible de conduire à une thrombose, sont alors minimisés.

Ce résultat positif est entre autre une conséquence du fait que le dispositif comporte des organes de maintien qui sont spécifiquement conçus pour maintenir les extrémités des conduits sur le dispositif. A cette innovation se rajoutent d'autres caractéristiques du dispositif, notamment la surface intérieure du dispositif, qui est particulièrement lisse afin d'éviter l'apparition des thromboses citées ci-dessus.

Une autre caractéristique de la présente invention consiste en l'utilisation d'un matériau résorbable et agissant en tant que promoteur de la reconstitution des tissus pour réaliser l'ensemble du dispositif. Le matériau se résorbe de façon contrôlée et surtout induit, dans un but curatif, la reconstitution des tissus en proposant un support favorable et en possédant des caractéristiques nécessaires pour une telle reconstitution. Ce caractère curatif d'induction d'une réparation des tissus peut encore être augmenté par des modifications spécifiques de la face externe du dispositif en fonction du type de tissu environnant le conduit circulatoire à réparer, afin de promouvoir la reconstitution cellulaire. Le maintien des conduits circulatoires connectés se fait donc au début par le dispositif lui-même et, après l'intervention chirurgicale, au fur et à mesure du temps nécessaire pour la cicatrisation voire reconstitution des tissus provoquée par le dispositif, par le tissu biologique propre à l'organisme du patient.

D'ailleurs, le geste chirurgical demandé pour la pose du dispositif est simplifié, accéléré et sécurisé par rapport au geste classique, dans le sens où aucune suture des extrémités du conduit circulatoire à réparer est nécessaire, le temps d'exécution du geste étant considérablement plus court et la reproductibilité d'un résultat positif étant élevée.

Le but de la présente invention est de réaliser un dispositif comportant les avantages énumérés ci-dessus.

Le dispositif pour connecter et maintenir et/ou protéger les conduits circulatoires se distingue à cet effet par les caractéristiques énumérées à la revendication 1 et/ou aux revendications dépendantes.

Les dessins annexés représentent, à titre d'exemple, une forme d'exécution et d'utilisation de l'invention.

La figure 1 est une représentation schématique d'une forme d'exécution du dispositif selon l'invention.

La figure 2 illustre schématiquement la mise en place et le maintien des conduits circulatoires sur le dispositif selon l'invention.

La figure 3 montre de façon schématique une extrémité de la paroi de l'élément cylindrique ou conique évidé selon une forme d'exécution du dispositif.

La figure 4 représente de façon schématique une partie du système de maintien du dispositif selon une forme d'exécution de l'invention.

Les figures 5a et 5b illustrent schématiquement deux possibilités d'agencement du système de maintien sur le dispositif selon l'invention.

L'invention sera maintenant décrite en détail en référence aux dessins annexés qui illustrent à titre d'exemple plusieurs formes d'exécution de l'invention.

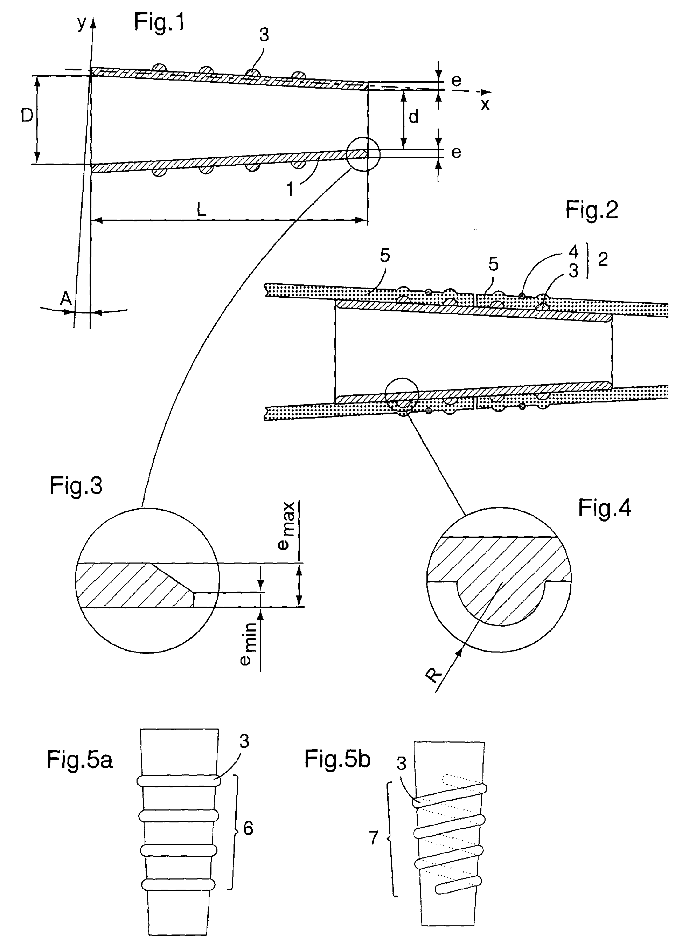

En référence à la figure 1, le dispositif comprend un élément de forme cylindrique ou conique évidé 1. Cet élément cylindrique ou conique évidé 1 étant le point principal du dispositif, il sera dans la suite du texte nommé aussi simplement dispositif, lorsque le contexte permet facilement la distinction.

Le dispositif se présente alors comme un tube cylindrique ou conique évidé 1, normalement destiné à jouer le rôle d'un connecteur, dont le diamètre ou les diamètres externes sont définis par les dimensions des conduits circulatoires à réparer.

Quand le dispositif comprend une seule valeur de diamètre externe, il prend la forme d'un connecteur cylindrique et peut être utilisé pour réaliser des anastomoses entre les deux parties d'un même vaisseau ou d'un même conduit sectionné pour les besoins de l'intervention chirurgicale ou encore deux vaisseaux ou deux conduits différents mais de même diamètre.

Quand le dispositif comprend plusieurs valeurs de diamètre externe, il prend la forme d'un connecteur conique et peut être utilisé pour réaliser des anastomoses entre deux vaisseaux ou deux conduits ayant des diamètres légèrement différents.

Les principaux paramètres déterminant le dispositif comme représenté schématiquement à la figure 1 sont le grand diamètre intérieur D, le petit diamètre intérieur d, la longueur hors tout L et l'épaisseur de la paroi e.

Les valeurs du grand diamètre extérieur Y = D + 2e et du petit diamètre extérieur y = d + 2e sont facilement accessibles.

La combinaison des paramètres d,Y et y détermine une grandeur supplémentaire appelée angle de cône du dispositif. Cette grandeur est repérée par la lettre A, exprimée en degré d'angles et déterminée par l'équation

$$A = arctg [ (Y-y) /2 \cdot L ]$$

Quand les valeurs de Y et y sont identiques, le dispositif est cylindrique. Dans ce cas, la valeur de l'angle de cône A est égale à zéro. Quand les valeurs de Y et y sont différentes, le connecteur est conique. Dans ce cas, la valeur de l'angle de cône A est non nulle.

L'épaisseur de la paroi e du dispositif peut être égale sur toute la longueur du dispositif ou peut prendre plusieurs valeurs variant entre les extrêmes $e_{min}$ et $e_{max}$, comme illustré dans la figure 3. Par exemple, les extrémités du dispositif pourront être effilées pour permettre un passage du liquide avec un minimum de perturbation. Une telle modification s'obtient en augmentant la valeur du diamètre intérieur sans varier la valeur du diamètre externe du dispositif (voir figure 3). L'épaisseur de la paroi du dispositif peut notamment aussi changer de manière à ce que la paroi de l'élément cylindrique ou conique évidé 1 comprenne une succession de renflements 3 et amincissements. Ceci est entre autre intéressant dans le cas où la restitution du tissu provoquée par le matériau résorbable du dispositif devrait se faire différemment, par exemple de façon non regulière, le long du dispositif.

La paroi de l'élément cylindrique ou conique évidé 1 est notamment caractérisée par sa surface lisse, surtout et de préférence concernant la surface intérieure de la paroi. Cette surface lisse permet d'éviter les thromboses.

Les renflements 3 disposés sur la surface externe de l'élément cylindrique ou conique évidé 1 du dispositif ont deux fonctions consistant à placer et à maintenir correctement en place le dispositif dans son logement.

En contact avec la paroi intérieure du conduit à réparer, les renflements 3 assurent, lors de la pose, une meilleure adhésion du dispositif sur les tissus à traiter en augmentant la surface de contact et en diminuant le coefficient de glisse du dispositif.

De préférence, les renflements 3 seront réalisés dans la masse du matériau constituant le dispositif. La forme extérieure des renflements 3 du dispositif est choisie pour éviter les angles et arêtes vives qui pourraient blesser la paroi interne du conduit à réparer, par conséquent une section de forme semi-circulaire (voir figure 4) convient particulièrement bien.

De façon préférentielle, mais non limitative, les renflements 3 peuvent être circulaires formant une série de couronnes 6, normalement en nombre pair, au minimum quatre, et en nombre identique sur chaque moitié du dispositif, disposées sur la longueur du dispositif (voir figure 5a) ou formant un bourrelet continu 7 décrivant une hélice sur la surface externe du dispositif (voir figure 5b).

La taille du renflement 3, caractérisée par la valeur R du rayon de la section circulaire du demi-disque, doit être correctement dimensionnée pour produire l'effet escompté. Une valeur trop petite pour le rayon R rend les renflements inefficaces, voire même inopérants, dans leur fonction. Par contre, une valeur trop élevée nuit à l'efficacité du dispositif ou peut engendrer des dommages sur le conduit circulatoire au moment de la ligature. Pour ces raisons, le rayon R prend généralement ses valeurs dans l'intervalle $(y/20) \leq R \leq (Y/5)$.

Dans les utilisations principales du dispositif, les valeurs attribuées aux paramètres mentionnés ci-dessus dépendront de l'application. Correspondant au domaine de l'application du dispositif, les valeurs (en mm ou °) des paramètres varient de la manière suivante:

- $1.0 \text{ mm} \leq Y \leq 70.0 \text{ mm}$ et $y \leq Y$,
- $(Y - y) \leq 2.0 \text{ mm}$,
- $5 \leq (L/Y) \leq 15$,
- $5 \leq (y/e_{Max}) \leq 20$,
- $A \leq 6°$

Le dispositif est alors réalisé dans une gamme de tailles par rapport au diamètre et à la longueur autorisant le traitement de plusieurs types de conduits circulatoires.

Par la suite, les renflements 3 offrent au chirurgien des emplacements privilégiés pour solidariser le conduit sur le dispositif par la pose d'un élément de maintien 4 et éviter que l'élément de maintien 4 ne glisse.

La nature de l'élément de maintien 4 des extrémités des conduits sur le dispositif sera choisie pour s'adapter au mieux à l'application en question. Cet élément de maintien sera le plus souvent constitué d'un lien ou d'un fil de suture compatible avec la spécialité chirurgicale concernée, mais il pourra aussi être spécifiquement confectionné. Dans ce dernier cas, l'élément de maintien sera le plus souvent constitué par un clip avantageusement réalisé dans le même matériau résorbable que le dispositif.

Suite à cette description, il est évident que le dispositif selon la présente invention comprend d'une part un élément cylindrique ou conique évidé 1 et d'autre part des organes de maintien 2 comportant selon la forme d'exécution de l'invention des renflements 3 sur la surface externe dudit élément et /ou un élément de maintien 4 comme par exemple un fil de suture ou des clips.

L'une des caractéristiques de l'invention consiste à utiliser un matériau résorbable pour la réalisation du dispositif. En effet, les matériaux résorbables connaissent plusieurs applications dans le domaine des dispositifs médicaux, par exemple en tant que fil de suture, ou en tant que prothèse, ou encore en tant que dispositif pour la libération contrôlée de substances médicamenteuses dans l'organisme. Ici, le matériau résorbable est destiné à être placé directement et intégralement dans un conduit circulatoire et à assurer, outre sa fonction principale d'élément réparateur ou protecteur, une fonction d'induction d'une action curative et évolutive provenant de l'organisme lui-même.

Les matériaux biodégradables trouvant des applications dans les domaines de la santé sont obtenus à partir de tissus ou de protéines provenant du règne animal, tels que le collagène ou le catgut, ou à partir de polymères produits par voie de synthèse.

Les natures chimiques des principaux polymères connus pour être résorbables regroupent les polyesters, les polyorthoesters, les polyanhydrides, les poly (éther) esters, les polyaminoacides et les polydepsipeptides (voir par exemple : B. Buchholz ; J. Mater. Sci. Mater. Med. **4** (1993) 381 - 388).

D'une façon plus schématique, mais non exhaustive, les polymères résorbables peuvent être décrits par un motif répondant à la formule générale :

-[-X1-C(O)-R1-Y1-R2-]-[-X2-C(O)-R3-Y2-R4-]-

dans laquelle :

C(O) désigne un groupement >C=O,
X1 ; X2 désignent un atome d'oxygène ou un groupement NH,
Y1 (respectivement Y2) désigne un atome d'oxygène, ou un groupement NH, ou une liaison chimique reliant directement R1 à R3 (respectivement R2 à R4),
R1 ; R2 ; R3 ; R4 désignent des chaînes carbonées linéaires ou ramifiées, saturées ou partiellement insaturées, portant ou non des hétéroatomes et contenant de 0 à 10 atomes de carbone.

Quand dans cette formule générale, X1 est égal à X2 et Y1 est égal à Y2 et R1 est égal à R3 et R2 est égal à R4, le polymère obtenu est appelé homopolymère. Dans le cas contraire, le polymère obtenu est appelé copolymère.

Parmi ces polymères, les inventeurs ont porté une attention toute particulière aux polymères pouvant être décrits par un motif répondant à la formule générale :

-[-X1-C(O)-R1-Y1-R2-]-[-X2-C(O)-R3-Y2-R4-]-

dans laquelle :

C(O) désigne un groupement >C=O,

X1 ; X2 désignent un atome d'oxygène,

Y1 (respectivement Y2) désigne un atome d'oxygène ou une liaison chimique reliant directement R1 à R3 (respectivement R2 à R4),

R1 ; R2 ; R3 ; R4 désignent des chaînes carbonées linéaires ou ramifiées et contenant de 0 à 5 atomes de carbone et de préférence de 0 à 3 atomes.

Ces types de polymères incluent par exemple les polylactides, les polyglycolides, les polydioxanones, les polyalkylènecarbonates et les polylactones. A ces homopolymères s'ajoutent encore les copolymères obtenus par combinaison des différents monomères.

Ces polymères sont connus pour leur aptitude à se résorber in vivo selon des modes de résorption connus et prévisibles.

En outre, parmi ces polymères, certains vont présenter des caractéristiques particulièrement intéressantes pour entrer dans la fabrication du dispositif selon la présente invention.

Ainsi, par exemple, les polydioxanones sont connues pour se résorber plus lentement que les polylactides, ou les polyglycolides, ou encore que le catgut ou le collagène.

D'un autre côté, la souplesse du matériau obtenu dépend également de la nature du polymère utilisé. Les caractéristiques mécaniques du matériau obtenu varieront par exemple avec la nature chimique du motif, le poids moléculaire, le procédé de polymérisation, la technique de mise en oeuvre du matériau, etc..

L'optimisation des différents paramètres influant sur les caractéristiques du matériau obtenu, et notamment son mode de résorption in vivo, a conduit à préférer les polydioxanones pour réaliser le dispositif. Les polydioxanones sont les polymères obtenus à partir de monomères cycliques répondant à la formule brute $C_4H_6O_3$ et possédant un groupement >C=O. Ils offrent une cinétique et un mode de résorption in vivo compatible avec les applications de l'invention.

Un traitement optionnel lors de la fabrication du dispositif permet de modifier sa surface externe pour augmenter son caractère d'induction de la reconstruction des tissus et motifs cellulaires. Ce traitement est fait en fonction du type de tissu autour des conduits circulatoires à réparer afin d'ajuster la surface externe du dispositif au mieux à l'endroit de l'implantation en question.

Cette modification peut intervenir par un traitement mécanique de la surface du polymère, consistant à créer un réseau de micro - perforations et/ou une immobilisation sur la surface du polymère d'une couche fonctionnalisée par des cellules du tissu à reconstruire ou par des macromolécules de type bioprotéines.

En ce qui concerne l'utilisation du dispositif, elle se distingue des techniques usuelles des anastomoses par la facilité de mise en place du dispositif, la rapidité de l'intervention et la reproductibilité élevée d'un résultat positif.

En utilisant le dispositif selon l'invention, la réalisation d'une anastomose entre les extrémités soit d'un seul conduit soit des conduits différents à traiter ou connecter consiste en quatre étapes principales.

Premièrement, la moitié de la longueur du dispositif, judicieusement sélectionné pour avoir un (ou des) diamètre(s) extérieur(s) proche(s) du (ou des) diamètre(s) intérieur(s) du (ou des) conduit(s) à réparer, est insérée dans l'une des extrémités. Ensuite, l'autre extrémité est enfilée sur la longueur du dispositif restée libre. Puis les deux extrémités sont positionnées pour qu'elles soient en contact et offrent une continuité apparente du (ou des) conduit(s) de manière à ce que le dispositif ne soit plus visible. Finalement, la mise en place du dispositif se complète par la pose d'éléments de maintien enserrant le (ou les) conduit(s) à réparer autour du dispositif par l'intermédiaire d'un système particulier. Comme mentionné ci-dessus, les organes de maintien 2 comprennent une série de renflements 3 disposés sur la surface externe du dispositif selon un arrangement prédéfini et des éléments de maintien 4 comme un fil ou des clips. Cet aspect de l'invention est fondamental dans la mesure où il assure le maintien post - implantation du (ou des) conduit(s) sur le dispositif pendant toute la durée nécessaire pour la cicatrisation.

Selon les habitudes du praticien, il peut être avantageux de recouvrir la surface externe du dispositif et/ou les extrémités des conduits sur lesquels sera pratiqué l'anastomose par une colle biologique.

En particulier, le mode de maintien des extrémités des conduits sur le dispositif utilisant un fil de suture chirurgical adapté à l'application procède généralement selon deux principes.

Dans le cas où le dispositif comporte des renflements de type "couronne", la ligature consiste à solidariser chaque segment de conduit au dispositif par une série de liens, ou fils de suture, disposés entre deux renflements successifs et noués.

Dans le cas où le dispositif comporte un renflement hélicoïdal, la ligature consiste à solidariser le conduit au dispositif par un lien, ou fil de suture, disposé dans la gorge de l'hélice et noué à chaque extrémité. Cette technique offre l'avantage de garantir une bonne homogénéité d'immobilisation des segments des conduits sur le dispositif.

En ce qui concerne les applications du dispositif, elles couvrent une large gamme de réparation ou de modification des conduits circulatoires comme applications principales.

Parmi ces applications principales du dispositif, utilisé en tant que connecteur, on citera à titre d'exemple les utilisations dans l'ensemble du domaine vasculaire périphérique, les utilisations dans la chirurgie de l'aorte et des artères qui en sont issues (thoraciques, abdominales et périphériques), les utilisations en chirurgie coronarienne et en chirurgie carotidienne, les utilisations en chirurgie digestive (par exemple, le connecteur peut être utilisé pour l'anastomose de deux segments intestinaux, sur tout le trajet intestinal de l'oesophage à l'anus), les utilisations en urologie.

Le dispositif trouve également des applications dans les anastomoses de conduits dans le but d'une refonctionnalisation (par exemple re-perméabilisation des trompes ou re-perméabilisation des canaux déférents).

Les applications secondaires du dispositif englobent les réalisations de passage au travers d'une membrane ou d'une cloison. Elles incluent également les protections ou renforcements de conduits pouvant être fragilisés, par exemple, lors de traitements agressifs.

La structure cylindrique ou conique du dispositif et son aptitude à la résorption spontanée autorisent par exemple des utilisations dudit dispositif pour ménager un passage perméable et temporaire à travers une membrane (par exemple à travers le tympan) ou des utilisations pour ménager un passage dans un but exploratoire (par exemple lors d'explorations endoscopiques), ou encore des utilisations de façon préventive pour protéger un conduit naturel traité de manière agressive par des voies physiques ou chimiques ou encore par l'emploi de radiations.

Le dispositif est destiné à être utilisé sur l'homme. Pour autant, les utilisations vétérinaires sont également possibles dans la mesure où les tailles disponibles pour le dispositif sont compatibles avec les conduits circulatoires à réparer chez l'animal.

**Revendications**

1. Dispositif pour la connexion et le maintien et/ou la protection des conduits des systèmes circulatoires comportant un élément de forme cylindrique ou conique évidé (1) en un matériau résorbable (bioabsorbable ou biodégradable), **caractérisé par le fait qu'**il comprend des organes de maintien (2) destinés à maintenir sans suture les extrémités des conduits des systèmes circulatoires à traiter sur ledit dispositif.

2. Dispositif selon la revendication 1, **caractérisé par le fait que** l'épaisseur de la paroi de l'élément cylindrique ou conique évidé (1) est constante le long dudit élément.

3. Dispositif selon la revendication 1, **caractérisé par le fait que** la paroi de l'élément cylindrique ou conique évidé (1) comporte une succession de renflements et/ou amincissements.

4. Dispositif selon l'une des revendications précédentes, **caractérisé par le fait que** les extrémités de la paroi de l'élément cylindrique ou conique évidé (1) sont effilées.

5. Dispositif selon la revendication 4, **caractérisé par le fait que** l'effilage des extrémités de la paroi est réalisé en augmentant le diamètre intérieur de l'élément cylindrique ou conique évidé (1) en gardant constant son diamètre extérieur.

6. Dispositif selon l'une des revendications précédentes, **caractérisé par le fait que** les organes de maintien (2) comportent des renflements (3) et/ou des amincissements situés sur la surface extérieure de l'élément cylindrique ou conique évidé (1).

7. Dispositif selon la revendication 6, **caractérisé par le fait que** les renflements (3) sont réalisés par une série de bourrelets formant des couronnes (6) de section semi-circulaire sur la surface extérieure de l'élément cylindrique ou conique évidé (1).

8. Dispositif selon la revendication 6, **caractérisé par le fait que** les renflements (3) sont réalisés par un bourrelet continu formant une hélice (7) de section semi-circulaire sur la surface extérieure de l'élément cylindrique ou conique évidé (1).

9. Dispositif selon l'une des revendications précédentes, **caractérisé par le fait que** les organes de maintien (2) comportent un (ou plusieurs) élément (s) de maintien (4) destiné(s) à maintenir les conduits des systèmes circulatoires (5) en place sur l'élément cylindrique ou conique évidé (1), de préférence en combinaison avec des renflements (3) et/ou amincissements sur la surface extérieure de cet élément cylindrique ou conique évidé (1).

10. Dispositif selon la revendication 9, **caractérisé par le fait que** les éléments de maintien (4) comportent des fils de suture.

11. Dispositif selon la revendication 9, **caractérisé par le fait que** les éléments de maintien (4) comportent des clips.

12. Dispositif selon l'une des revendications précédentes, **caractérisé par le fait que** l'élément cylindrique ou conique évidé (1) du dispositif est réalisé dans un polymère pouvant être décrit par le motif général -[-X-C(O)-R1-Y-R2-]- dans lequel C(O) désigne un groupement >C=O ; X désigne un atome d'oxygène ; Y désigne un atome d'oxygène ou une liaison chimique reliant R1 à R2 ; R1 et R2 désignent des chaînes carbonées linéaires ou ramifiées et contenant de 0 à 5 atomes de carbone et de préférence de 0 à 3 atomes.

13. Dispositif selon la revendication 12, **caractérisé par le fait que** le polymère utilisé est de type polylactide, ou polyglycolide, ou polylactone, ou polyalkylènecarbonate, ou encore de préférence de type polydioxanone ou de tout autre type de polymère pourvu que ce dernier soit obtenu à partir d'un composé cyclique répondant à la formule brute $C_4H_6O_3$ et possédant un groupement >C=O.

**14.** Dispositif selon l'une des revendications 1 à 11, **caractérisé par le fait que** le polymère utilisé pour réaliser l'élément cylindrique ou conique évidé (1) du dispositif est un copolymère obtenu par combinaison des différents monomères conduisant aux polymères revendiqués dans les revendications 12 et 13.

**15.** Dispositif selon l'une des revendications précédentes, **caractérisé par le fait que** les renflements (3) situés sur la surface extérieure de l'élément cylindrique ou conique évidé (1) sont réalisés en un matériau résorbable (bioabsorbable ou biodégradable).

**16.** Dispositif selon la revendication 15, **caractérisé par le fait que** le matériau résorbable (bioabsorbable ou biodégradable) utilisé pour réaliser les renflements (3) est identique à celui utilisé pour réaliser l'élément cylindrique ou conique évidé (1).

**17.** Dispositif selon l'une des revendications précédentes, **caractérisé par le fait que** les éléments de maintien (4) sont réalisés en un matériau résorbable (bioabsorbable ou biodégradable).

**18.** Dispositif selon la revendication 17, **caractérisé par le fait que** le matériau résorbable (bioabsorbable ou biodégradable) utilisé pour réaliser les éléments de maintien (4) est identique à celui utilisé pour réaliser l'élément cylindrique ou conique évidé (1).

**19.** Dispositif selon l'une des revendications précédentes, **caractérisé par le fait que** la surface intérieure de l'élément cylindrique ou conique évidé (1) est lisse.

**20.** Dispositif selon l'une des revendications précédentes, **caractérisé par le fait que** la surface extérieure du dispositif est conformée afin d'augmenter l'induction de la reconstitution des tissus.

**21.** Dispositif selon la revendication 20, **caractérisé par le fait que** la surface extérieure du dispositif comporte des micro-perforations de la couche de polymère.

**22.** Dispositif selon la revendication 20, **caractérisé par le fait que** la surface extérieure du dispositif comporte une couche de cellules de même nature que celle du tissu à reconstruire.

**23.** Dispositif selon la revendication 20, **caractérisé par le fait que** la surface extérieure du dispositif comporte une couche de macromolécules de types protéines.

**24.** Dispositif selon l'une des revendications précédentes, **caractérisé par le fait que** le dispositif est enduit de colle biologique.

**25.** Utilisation d'un dispositif selon les revendications 1 à 24, pour laquelle l'intervention chirurgicale sera pratiquée sur un (ou des) conduit(s) du système circulatoire sanguin.

**26.** Utilisation d'un dispositif selon les revendications 1 à 24, pour laquelle l'intervention chirurgicale sera pratiquée sur un (ou des) conduit(s) du système intestinal.

**27.** Utilisation d'un dispositif selon les revendications 1 à 24, pour laquelle l'intervention chirurgicale sera pratiquée sur un (ou des) conduit(s) du système génital.

**28.** Utilisation d'un dispositif selon les revendications 1 à 24, pour laquelle l'intervention chirurgicale sera pratiquée sur un (ou des) conduit(s) du système urinaire.

Fig.1

Fig.2

Fig.3

Fig.4

Fig.5a

Fig.5b

EP 1 275 344 A1

**Office européen des brevets**

# RAPPORT DE RECHERCHE EUROPEENNE

Numéro de la demande

EP 01 11 6534

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 6 056 762 A (NASH) 2 mai 2000 (2000-05-02) | 1-4,6, 9-19 | A61B17/11 A61F2/06 |
| Y | * colonne 5, ligne 50 - colonne 6, ligne 38; figures 4,7 * | 22 | |
| X | WO 94 13356 A (AVATAR) 23 juin 1994 (1994-06-23) * figure 5 * | 1,20,21 | |
| X | DE 36 05 306 A (EULER) 20 août 1987 (1987-08-20)  * colonne 4, ligne 31 - ligne 54; figures 3-5 * | 1-3,6,9, 11-13, 17,19 | |
| X | US 5 376 376 A (LI) 27 décembre 1994 (1994-12-27)  * colonne 7, ligne 51 - ligne 60 * * colonne 10, alinéa 5 - alinéa 7 * | 1,4,5, 20,21, 23,24 | |
| X | US 5 865 723 A (LOVE) 2 février 1999 (1999-02-02) * figures 7-11 * | 1,8 | DOMAINES TECHNIQUES RECHERCHES (Int.Cl.7) A61B A61F |
| Y | WO 98 14135 A (U.MINNESOTA) 9 avril 1998 (1998-04-09) * page 9, ligne 14 - page 11, ligne 18; figure 3 * | 22 | |

Le présent rapport a été établi pour toutes les revendications

| Lieu de la recherche | Date d'achèvement de la recherche | Examinateur |
|---|---|---|
| LA HAYE | 10 décembre 2001 | Barton, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**   EP 01 11 6534

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-12-2001

| Document brevet cité au rapport de recherche | | Date de publication | Membre(s) de la famille de brevet(s) | | Date de publication |
|---|---|---|---|---|---|
| US 6056762 | A | 02-05-2000 | AU | 7250098 A | 11-12-1998 |
| | | | EP | 0983025 A1 | 08-03-2000 |
| | | | WO | 9852474 A1 | 26-11-1998 |
| | | | US | 6030395 A | 29-02-2000 |
| | | | US | 6036705 A | 14-03-2000 |
| WO 9413356 | A | 23-06-1994 | US | 5425739 A | 20-06-1995 |
| | | | AU | 5743694 A | 04-07-1994 |
| | | | WO | 9413356 A1 | 23-06-1994 |
| DE 3605306 | A | 20-08-1987 | DE | 3605306 A1 | 20-08-1987 |
| | | | WO | 8704915 A1 | 27-08-1987 |
| | | | EP | 0263122 A1 | 13-04-1988 |
| US 5376376 | A | 27-12-1994 | CA | 2199951 A1 | 21-03-1996 |
| | | | WO | 9608222 A1 | 21-03-1996 |
| | | | US | 5512291 A | 30-04-1996 |
| | | | US | RE36370 E | 02-11-1999 |
| | | | EP | 0783285 A1 | 16-07-1997 |
| | | | JP | 10505527 T | 02-06-1998 |
| US 5865723 | A | 02-02-1999 | AU | 720362 B2 | 01-06-2000 |
| | | | AU | 1569297 A | 28-07-1997 |
| | | | CA | 2240989 A1 | 10-07-1997 |
| | | | EP | 0874603 A1 | 04-11-1998 |
| | | | JP | 2000502586 T | 07-03-2000 |
| | | | WO | 9724081 A1 | 10-07-1997 |
| WO 9814135 | A | 09-04-1998 | US | 6057137 A | 02-05-2000 |
| | | | WO | 9814135 A1 | 09-04-1998 |

Pour tout renseignement concernant cette annexe : voir Journal Officiel de l'Office européen des brevets, No.12/82